# EUROPÄISCHE PATENTANMELDUNG

(11) **EP 1 743 595 A2**
(43) Veröffentlichungstag der Anmeldung: **17.01.2007**
(21) Anmeldenummer: 06021589.4
(22) Anmeldetag: 29.05.2000
(51) Int. Cl.: A61C 8/00

(54) **Verwendung einer medizinischen Membran zur Paradontalregeneration**

(62) Teilanmeldung aus: 00927664.3
(71) Anmelder: CelGen AG, 6300 Zug (CH)
(72) Erfinder: Die Erfindernennung liegt noch nicht vor
(74) Vertreter: Schrell, Andreas

(57) **Zusammenfassung**

Die vorliegende Erfindung stellt Zugbefestigungsvorrichtungen und medizinische Membranen zur Knochenregeneration zur Verfügung, die es erlauben, Kallusdistraktion zwischen Knochen und medizinischer Membran zu erzeugen. Die medizinische Membran besitzt mindestens eine Oberfläche, die mindestens ein Befestigungselement für ein Zugmittel (6) besitzt. Die Zugbefestigungsvorrichtung zur Knochenregeneration umfasst ein Befestigungsmittel, ein einstellbares Spannmittel und ein Zugmittel (6) zur Befestigung der Membran. Mittels der Zugspannvorrichtung wird eine einstellbare Zugspannung auf die Membran ausgeübt, was Knochenregeneration induziert.

## Beschreibung

### Hintergrund

Die vorliegende Erfindung betrifft Vorrichtungen und medizinische Membranen zur aktiven Knochenregeneration mittels Kalltisdistraktion gegen eine künstliche Grenzfläche, gemäss dem Oberbegriff der unabhängigen Ansprüche.

### Stand der Technik

Die Kallusdistraktion ist bereits seit über 100 Jahren bekannt und ist heute zur Therapie der ersten Wahl geworden. Der wichtigste biologische Reiz für die Knochenbildung ist die mechanische Beanspruchung. Dadurch werden piezoelektrische Kräfte freigesetzt, die Osteoblasten und Osteoklasten aktivieren. Die Distraktionsosteogenese induziert die Knochenneubildung, indem durch langsame Trennung von Knochensegmenten biologische Wachstumsreize ausgelöst werden. Durch diese Methode wird die direkte Bildung von Geflechtsknochen durch Distraktion erreicht. Die definierte Zugspannung bei der Knochenregeneration ist wesentlich und wird augenscheinlich, wenn sie mit zueinander fixierten Knochen bei Defektüberbrückung verglichen wird. Bereits bei 5 mm Ausdehnung können Knochendefekte nicht suffizient überbrückt werden. Legt man an die Knochenfragmente eine definierte Zugspannung an, so gewinnt das mesenchymale Gewebe im Spalt und an den angrenzenden Fragmentenden sein osteogenetisches Potential zurück. Bei Vorliegen ausreichender vaskulärer Potenz kommt es unter progressiver Distraktion zur Metaplasie des organisierten Hämatoms in einer Zone von longitudinal arrangiertem, fibrösem Gewebe, das sich unter optimalen externen und internen Bedingungen direkt in Geflechtsknochen umwandeln kann. Im Zeitverlauf zeigt sich, dass nach Abschluss der Distraktion keine wesentliche Zunahme der gesamten gebildeten Knochenmasse entsteht. Die Rate und die Frequenz der Distraktion ist wichtig. Nach einer folgenden Retentionsphase von 6 Wochen mit nochmaligen 6 Wochen Konsolidierungsphase ist die Osteogenese komplett abgeschlossen. Die Distraktions-Osteogenese ist zur Zeit die innovativste chirurgische Technik auf den Gebieten der Orthopädie, Traumatologie und Mund-, Kiefer und Gesichtschirurgie.

Die Verwendung medizinischer Membranen bei der gesteuerten Geweberegeneration entwickelte sich aus der Wundheilung. Die therapeutischen Konzepte GTR (Guided Tissue Regeneration) und GBR (Guided Bone Regeneration) basieren auf der Überlegung, dass während des Heilungsprozesses im Wundgebiet ansässige Zelltypen mit unterschiedlicher Wachstumsrate durch Zwischenschaltung von speziellen Membranen selektiv beeinflusst werden. Unerwünschte Zellformen mit höherer Wachstumsrate werden abgeschirmt und die Besiedelung des Defekts mit Zellen, die das gewünschte Gewebe neu bilden können, gefördert. Mit der GTR.Technik werden schnellwachsende Epithelzellen und Bindegewebszellen gehemmt und die langsamwachsenden Knochenzellen übernehmen ungestört die Defektheilung. Obwohl die Verwendung spezieller Membranen in den paradontalen Regenerationstechniken die Bildung von neuem Knochen sowie Bindegewebe ermöglicht, sind die Erfolge begrenzt. Diese Methoden erlauben nur begrenzte Apposition, da über die Membran kein biomechanischer Reiz auf den Knochen übertragen wird d.h. es wird weder Zug noch Druck auf die Membran ausgeübt.

Allen bisherigen Techniken der Distraktionsosteogenese ist gemeinsam, dass der gebildete Kallus zwischen zwei Knochenfragmenten gedehnt wird. Der Erfindung liegt daher die Aufgabe zugrunde Mittel zur Verfügung zu stellen, die Knochenregeneration zwischen Kallus und künstlicher Grenzfläche erlauben.

### Darstellung der Erfindung

Diese Aufgabe wird von den unabhängigen Ansprüchen der vorliegenden Erfindung gelöst. Die vorliegende Erfindung stellt Zugbefestigungsvorrichtungen und medizinische Membranen zur Verfügung, die es erlauben Kallusdistraktion zwischen Knochen und medizinischer Membran zu erzeugen, indem auf die Membran über ein mit der Zugbefestigungsvorrichtung verbundenes Zugelement eine Zugspannung erzeugt wird. Die Membran ist mit dem Kallus verwachsen und deren dosierte Bewegung induziert Knochenneubildung. Die Gegenstände der vorliegenden Erfindung finden Anwendung z.B. in der Zahnmedizin bei paradontaler Regeneration, im Kieferkammaufbau in der Implantalogie oder ästhetischen Gesichtschirugie, in der Orthopädie und Traumatologie.

Die vorliegende Erfindung stellt eine medizinische Membran zur Knochenregeneration zur Verfügung. Die Membran weist mindestens eine Oberfläche mit Befestigungselementen für ein Zugmittel und/oder Verstärkungspunkte auf. Als Befestigungselemente sind z.B. Ösen, Löcher in der Membran, Ansatzpunkte auf der Membran verwendbar. Die Befestigungselemente ermöglichen ein Anbringen eines Zugmittels zur Erzeugung einer einstellbaren Zugspannung auf die Membran. Die Verstärkungspunkte ermöglichen die Kraftübertragung auf die Membran, die z.B. mittels Anhebung eines unter der Membran liegenden Hebemittels in der Distraktionsrichtung bewegt wird. Die von unten auf die Membran übertragene Kraft übt auf den unter der Membran liegenden und mit dieser verbundenen Knochen wiederum Zug aus, was zur Induktion von Knochenregeneration führt. Als Verstärkungspunkte sind z.B. dichter ausgestaltete Teile der Membran, ausgewählte Punkte auf den Verstärkungsleisten verwendbar.

Vorteilhaft besitzt mindestens eine Oberfläche der Membran Verstärkungsleisten, die eine gleichmässige Kraftübertragung auf eine erfindungsgemässe Membran ermöglichen. In einer bevorzugten Ausführungsform wird die gleichmässige Kraftübertragung auf die Membran mittels chemischer Oberflächenhärtung erreicht.

Vorteilhaft ist die dem Knochen zugewandte Oberfläche aufgerauht, insbesondere porös, um eine Verankerung des Kollagens und ein Einwachsen der Osteoblasten zu ermöglichen. Das Zellattachment und die Verankerung von Kollagen des darunterliegenden Knochens mit der Knochen zugewandten Seite der Membran führt zu einer Verankerung der Membran im Kallus. Dies erlaubt, dass auf die Membran Zugspannung ausgewirkt werden kann, ohne die Membran vom Untergrund abzulösen. In einer bevorzugten Ausführungsform besteht die Knochen zugewandte Seite der erfindungsgemässen Membran aus einem nicht resorbierbaren Material z.B. Titan. In einer weiteren bevorzugten Ausführungsform besteht die dem Knochen zugewandte Oberfläche aus einem resorbierbaren Material, bevorzugter aus Kollagen mit einer Maschenweite von 20-100µm.

Die von Bindegewebe bedeckte, äussere, Oberfläche der Membran ist bevorzugt aufgerauht, um eine Verankerung des bedeckenden Bindegewebes zu erlauben. Die aufgerauhte Oberfläche ist bevorzugt porös ausgestaltet, um ein Attachement der Bindegewebszellen und eine Verankerung des Kollagens zu erlauben. Diese Ausgestaltung verhindert ein Ablösen des Bindegewebes, wenn die Membran bewegt wird. Die Oberflächen der heute verwendeten Membranen sind so beschaffen, dass eine Bewegung der Membranen zum leichten Ablösen des umgebenden Bindegewebes führt. Die Ablösung des Gewebes von der Membranoberfläche führt zur Einwanderung von Bakterien in den entstandenen Spalt und zu Infektionen. Die Oberflächenbeschaffenheit der erfindungsgemässen Membran erlaubt nun eine Bewegung der Membran ohne Ablösung des umgebenden Bindegewebes. Diese Eigenschaften der erfindungsgemässen Membran erschliesst dieser neue Anwendungsgebiete in der Medizin wie z.B. Induktion von Knochenregeneration durch Übertragung eines biomechanischen Reizes auf einen Knochen über eine Membran.

Vorteilhaft ist die Membran für Serum durchlässig, um eine Ernährung des umgebenden Gewebes zu erlauben. Vorteilhaft ist die Membran undurchlässig für Epithel- und Bindegewebszellen, um eine Einwanderung konkurrierender Zellen ins Regenerationsgebiet zu verhindern.

Bevorzugt ist eine Membran, die formstabil ist. Unter formstabiler Membran wird eine Membran verstanden, die ihre Form bei Belastung mit dem Eigengewicht im wesentlich beibehält. Als Zugmittel sind beispielsweise brauchbar Fäden aus z.B. Polypropylen, Polyester, Polyamid und Drahtligaturen.

Die Membran kann aus resorbierbaren wie auch aus nicht resorbierbaren Materialen hergestellt werden. Die resorbierbaren Materialen sind so beschaffen, dass sie ein Bestehen der Membran während der Distraktions-, Retentions- und Konsolidierungsphase der Knochenregeneration gewährleisten. Die Distraktionsphase dauert ungefähr 2-6 Wochen, die Retentionszeit hat ungefähr eine Dauer von 6 Wochen und die Konsolidierungsphase dauert ungefähr 6 Wochen.

Bevorzugt sind Membranen, die eine dreidimensionale Fläche bilden und segmentiert sind. Unter segmentierter Membran wird verstanden, dass die einzelnen Segmente der Membran gelenkig miteinander verbunden sind.

Die segmentierte Ausgestaltung der Membran erlaubt bei einem grossen zu behandelnden Knochenabbau eine unterschiedliche Hebung der unterschiedlichen Segmente und ermöglicht somit eine optimale Anpassung bei grossflächigen, unterschiedlichen Defekten. Vorteilhaft ist die erfindungsgemässe Membran elastisch, um grosse Anpassung an die verschiedenen Gegebenheiten zu erlauben.

Die erfindungsgemässen Membranen weisen verschiedene Formen auf. Die verschiedenen Ausgestaltungen der Membranen gewährleisten eine optimale Anpassung an die Gegebenheiten.

Die Membranen werden z.B. bei der Verwendung in der paradontalen Knochenregeneration durch Lappenbildung, horizontale Periostschlitzung und exakter Adaptation der Membran operativ dem Patienten eingesetzt. Nach Adaptation der Membran wird das Zugmittel an den Befestigungselementen der Membranoberfläche eingefädelt und die Wunde geschlossen.

Ein anderer Gegenstand der vorliegenden Erfindung ist eine Zugbefestigungsvorrichtung zur Knochenregeneration, die mittels eines Zugmittels mit der implantierten medizinischen Membran verbunden werden kann, um eine Zugspannung auf die Membran auszuüben. Die Zugbefestigungsvorrichtung umfasst ein Befestigungsmittel und ein einstellbares Spannmittel, mit dem das Zugmittel verbunden ist und z.B. durch Drehung eine dosierbare Zugspannung auf-die Membran bewirkt. Als Befestigungsmittel sind beispielsweise Grundplatten brauchbar und als Spannmittel sind beispielsweise Bolzen, Schieber, Zahnräder und Spulräder brauchbar. Das Zugmittel kann beispielsweise durch Aufwicklung oder translatorische Hebung des Spannmittels Spannung auf die verbundene Membran übertragen.

Die Erfindung bezieht sich auf ein Verfahren zur Behandlung eines Knochendefekts im Zahnbereich, wobei eine erfindungsgemässe Membran auf ein zu regenerierendes Knochensegment appliziert wird und auf diese Membran über eine Zugbefestigungsvorrichtung eine Zugspannung ausgeübt wird.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Figur 1 einen Schnitt durch eine beispielhafte Anordnung der vorliegenden Erfindung bei Verwendung zur Knochenregeneration im Zahnbereich,
Figur 2 eine Explosionszeichnung eines Ausführungsbeispiels der Zugbefestigungsvorrichtung,
Figur 3 eine Aufsicht des gleichen Ausführungsbeispiels der Figur 2,
Figur 4 eine Seitenansicht des Ausführungsbeispiels der Figuren 2 und 3,
Figur 5 eine Aufsicht eines weiteren beispielhaften Ausführungsbeispiels der Zugbefestigungsvorrichtung,
Figur 6 eine Seitenansicht des Ausführungsbeispiels von Figur 5,
Figur 7 eine Seitenansicht eines Ausführungsbeispiels der Zugbefestigungsvorrichtung,
Figur 8 eine Aufsicht eines weiteren Ausführungsbeispiels der Zugbefestigungsvorrichtung,
Figur 9 zeigt eine beispielhafte Ausführungsform einer Membran mit gehärteter Oberfläche,
Figur 10 zeigt die Membran der Figur 9 in applizierter Form zwischen zwei Zähnen,
Figur 11 zeigt die Membran der Figur 9 in applizierter Form an mehreren Zähnen,
Figur 12 zeigt eine beispielhafte Ausführungsform einer Membran mit Verstärkungsleisten,
Figur 13 zeigt die Membran der Figur 12 in applizierter Form zwischen zwei Zähnen,
Figur 14 zeigt die Membran der Figur 12 in applizierter Form an mehreren Zähnen,
Figur 15 zeigt eine beispielhafte Ausführungsform einer segmentierten Membran an Zähnen,
Figur 16 zeigt eine beispielhafte Anordnung einer erfindungsgemässen Membran zur Knochenregeneration mittels Anhebung durch z.B. provisorische Implantate und
Figur 17 einen Querschnitt durch eine bespielhafte dreichschichtige Membran mit zwei aufgerauhten Oberflächen.

### Wege zur Ausführung der Erfindung

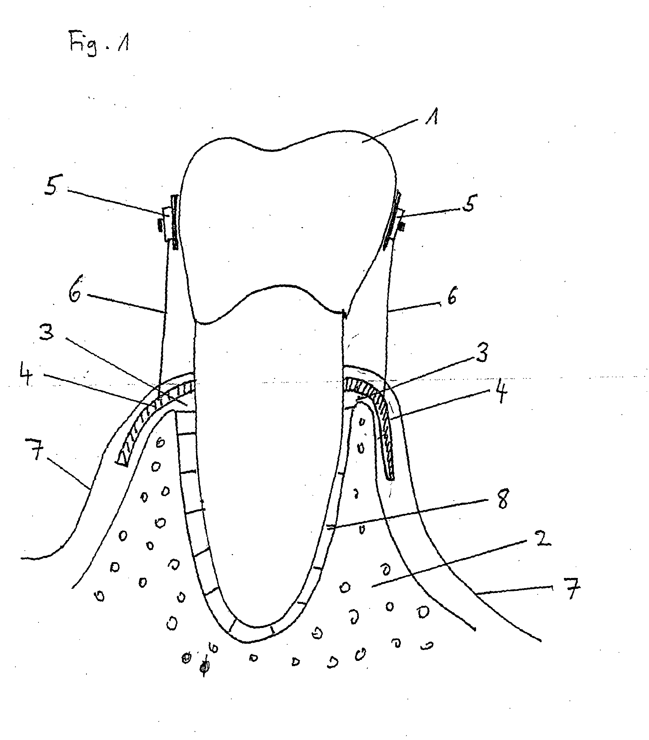

Die Figur 1 zeigt eine Anordnung der erfindungsgemässen Membran und der erfindungsgemässen Zugbefestigungsvorrichtung bei Verwendung zur Knochenregeneration im Zahnbereich. In der Übersichtsfigur 1 ist ein Querschnitt eines Zahnes 1 dargestellt, der den Knochen 2, eine Läsion des paradontalen Gewebes 3, eine erfindungsgemässe Membran 4, ein Zugmittel 6 und eine erfindungsgemässe Zugbefestigungsvorrichtung 5, das Zahnfleisch 7 sowie das Paradontium 8 zeigt.

Die erfindungsgemässe Membran 4 wird über das Zugmittel 6 mit der Zugbefestigungsvorrichtung 5 verbunden und durch z.B. aufrollen des Zugmittels 6 wird eine Zugspannung auf die Membran 4 ausgeübt. Die erfindungsgemässe Membran 4 überträgt den biomechanischen Reiz auf den darunter liegenden Knochen 2, was zu Paradontalregeneration führt, welche die Regeneration des Knochens, des paradontalen Ligaments, der Wurzelhaut, der Gingiva und der Papillen umfasst.

Im folgenden werden Ausführungsbeispiele der Erfindung anhand der Zeichnungen näher erläutert.

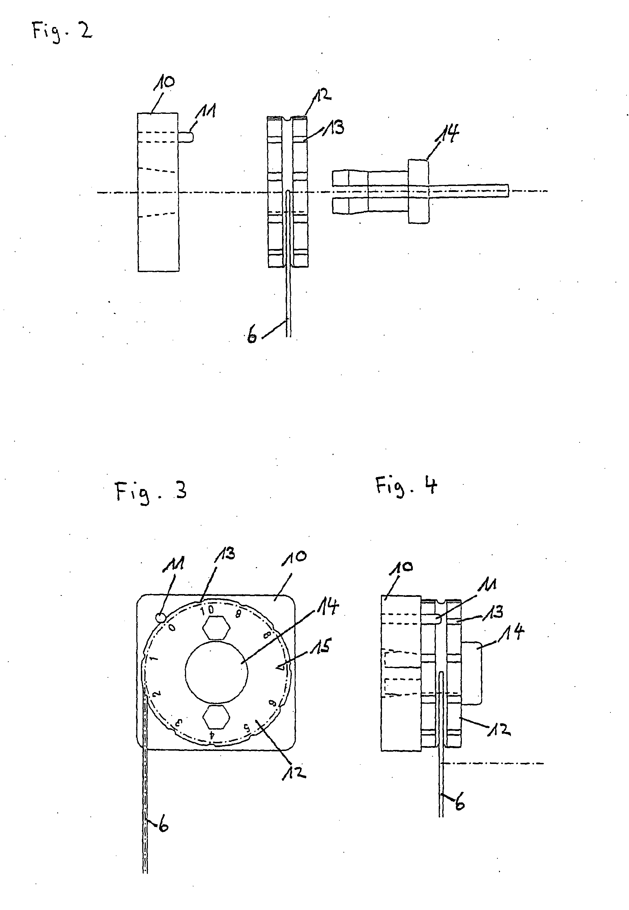

Figur 2 zeigt eine beispielhafte Ausführungsform der Zugbefestigungsvorrichtung in Seitenansicht. Die Zugbefestigungsvorrichtung umfasst eine Grundplatte 10 mit einem flexiblen Arretierstift 11 und eine im wesentlichen flache Auflagefläche 16, ein Spulrad 12 mit Kerben 13 und einen Spreizstift 14. Das Spulrad 12 ist mittels des Spreizstifts 14 drehbar auf der Grundplatte 10 gelagert. Der flexible Arretierstift 11 schnappt in eine Kerbe 13 des Spulrades 12 ein und fixiert dieses in einer definierten Stellung. Am Spulrad 12 ist das Zugmittel 6 befestigt.

Figur 3 zeigt die Ausführungsform der Figur 2 als Aufsicht. Das Spulrad 12 ist in Ausgangsstellung dargestellt, in der die Ziffer "0" der Skala 15 des Spulrades 12 mit dem Arretierstift 11 übereinstimmt. Durch Drehen auf die nächst höhere Ziffer der Skala 15 des Spulrads 12 wird das Zugmittel 6 um jeweils 1 mm zusätzlich angehoben. Das Spulrad ist auf einen Gesamthub von 10 mm ausgelegt.

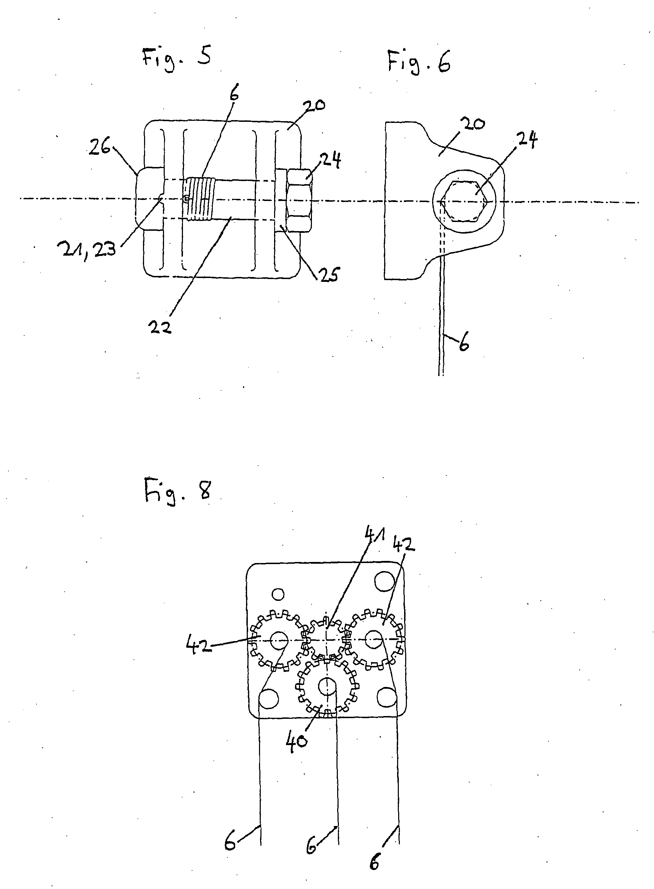

Figur 4 zeigt die Ausführungsform der Figur 2 in Seitenansicht. Die im wesentlichen flache Ausgestaltung der Auflagefläche 16 der Grundplatte 10 ist sichtbar. Die im wesentlichen flache Ausgestaltung der Auflagefläche 16 erlaubt eine adhäsive Befestigung der Zugbefestigungsvorrichtung z.B. an einem,Zahn.

Eine weitere beispielhafte Ausführungsform der Zugbefestigungsvorrichtung ist in Figuren 5 und 6 dargestellt.

Figur 5 zeigt eine Aufsicht der Ausführungsform. Die Vorrichtung umfasst eine Grundplatte 20 mit einer Nase 21, einem Wickelbolzen 22, der mit mehreren Nuten 23 versehen ist, eine Sechskantmutter 24 sowie eine elastische Unterlegscheibe 25 und das Zugmittel 6. In der Ausgangstellung rastet die Nase der Grundplatte 21 in die Nut 23 des Wickelbolzens 22 ein. Bei Drehung des Wickelbolzens 22 hebt sich der Kopf des Wickelbolzens 26 ab. Diese Längsbewegung wird durch die elastische Unterlegscheibe 25 aufgenommen. Diese stellt auch sicher, dass der Wickelbolzen 22 beim Weiterdrehen in der nächsten Nut 23 einrastet. Durch die Anzahl der Nuten und die entsprechende Dimensionierung des Wickelbolzendurchmessers wird ein exakt definierter Fadenhub pro Teildrehung erreicht.

Figur 6 stellt eine Seitenansicht der in Figur 5 dargestellten Ausführungsform dar. Die im wesentlichen flache Ausgestaltung der Auflagefläche 27 der Grundplatte 20 ist sichtbar.

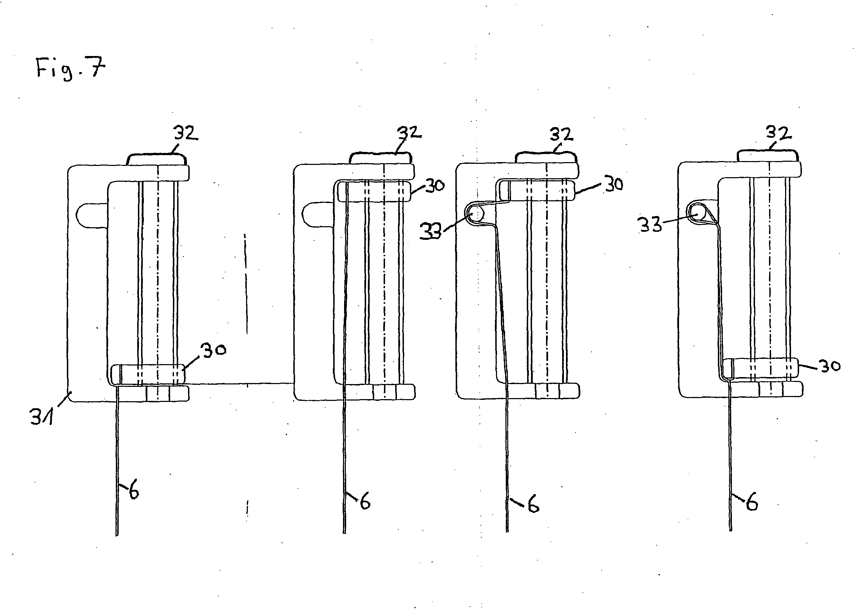

Figur 7 stellt eine weitere Ausführungsform in Seitenansicht dar. In diesem erfindungemässen Ausführungsbeispiel wird die Anhebung des Zugmittels 6 durch einen Schieber 30 bewerkstelligt, der sich in einer Grundplatte 31 bewegt. Der Schieber 30 wird durch Rechtsdrehung einer Schraube 32 von der unteren Ausgangsstellung in die obere Endlage bewegt. Mittels Umlenkung des Zugmittels 6 um einen Stift 33 wird ein zusätzlicher Fadenhub erreicht. Durch Drehen der Schraube 32 im Gegenuhrzeigersinn wird der Schieber 30 in die untere Ausgangsstellung zurück bewegt. Durch die Umlenkung des Fadens um den Stift 33 ergibt sich bei der Abwärtsbewegung nur der halbe Fadenhub pro Umdrehung der Schraube 32. Der insgesamte Fadenhub dieser Vorrichtung beträgt 7.7 mm.

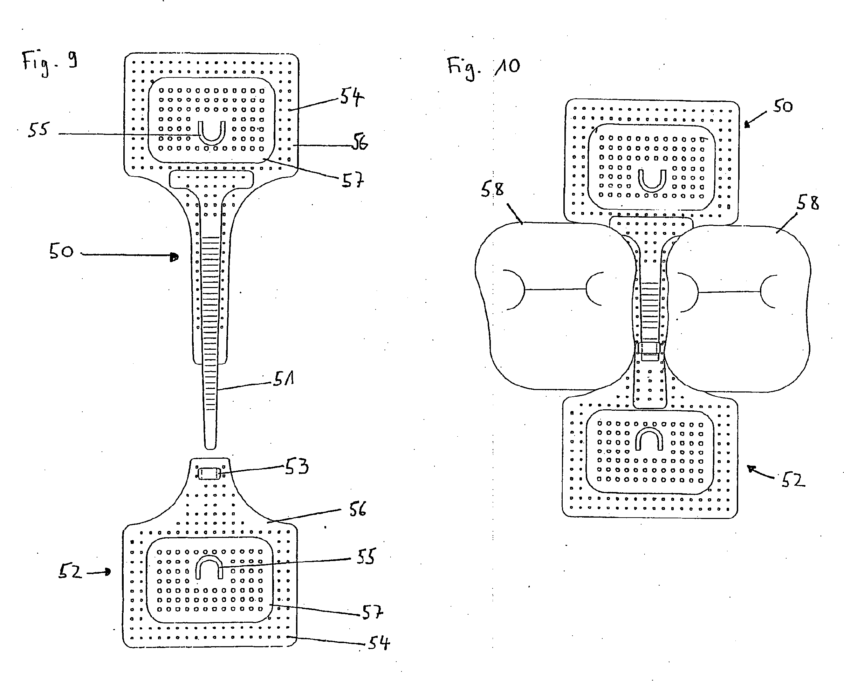

In Figur 8 ist eine Aufsicht einer weiteren Ausführungsform dargestellt, wobei durch Drehen eines Zahnrades 40 über ein Zahnrad 41 zwei Zahnräder 42 in Drehung versetzt werden. Die Achsen der Zahnräder 42 und des Zahnrades 40 wickeln ihrerseits gleichzeitig mehrere Zugmittel 6 auf. Diese Ausführungsform ermöglicht eine Spannungsübertragung über mehrere Zugmittel.

In einer bevorzugten Ausführungsform weisen die Zugbefestigungsvorrichtungen der vorliegenden Erfindung eine Grösse auf, die eine Befestigung an einem Zahn erlaubt.

Allen beschriebenen Ausführungsbeispielen ist gemeinsam, dass sie einen genau einstellbaren Fadenhub erlauben und somit eine dosierte Zugspannung auf die mit der Vorrichtung verbundenen medizinische Membran ausgeübt werden kann. Die genaue Dosierung der Zugspannung ist für die Knochenregeneration wesentlich, da bei einer Überdehnung unerwünschte Gewebeveränderungen entstehen und nicht das gewünschte Gewebe regeneriert wird.

Die Zugbefestigungsvorrichtungen können z.B. durch die Säureätztechnik am bukkalen und lingualen Schmelz des Zahnes oder in einem vorher angebrachten Bracket befestigt werden. Zur Befestigung der Vorrichtungen eignen sich z.B. auch Implantate, provisorische Implantate, Schienen und Kunststoffprothesen.

Eine Anordnung bestehend aus einer erfindungsgemässen Zugbefestigungsvorrichtung und einer medizinischen Membran ermöglicht eine vollständige Regeneration von paradontalen Defekten. Sie erlaubt die Übertragung mechanischer Reize auf den unter der Membran liegenden Kallus, was Knochenneubildung anregt. Dies ergibt völlig neue Perspektiven in der Behandlung von Paradontopathien. Sowohl das umgebende Weichgewebe als auch der Alveolarknochen werden auf das ursprüngliche Niveau rekonstruiert. Selbst grossflächige Defekte können durch Verwendung der erfindungsgemässen Anordnung rekonstruiert werden.

Die Figuren 9-15 stellen beispielhafte Ausführungsformen erfindungsgemässer Membranen dar.

Figur 9 zeigt ein Ausführungsbeispiel einer oberflächengehärteten Membran. Die Membran besteht aus einem Teil 50 mit Lasche 51 und einem Teil 52 mit Durchtrittsöffnung 53 für die Lasche 51. Beide Teile der beispielhaften Ausführungsform besitzen Poren 54 und Befestigungselemente 55 zur Befestigung eines Zugmittels 6. Die Randzonen 56 der beiden Teile der Membran 50,52 sind weich ausgebildet, um ein dichtes, enges Anliegen um die Zahnwurzel zu erlauben. Die zentrale Zone 57 der beiden Teile 50,52 der Membran ist mittels chemischer Verfahren ausgehärtet, um eine gleichmässige Verteilung der Zugkraft auf die gesamte Membran zu ermöglichen. In einer bevorzugten Ausführungsform erfolgt die Aushärtung der Membran während der Operation in situ.

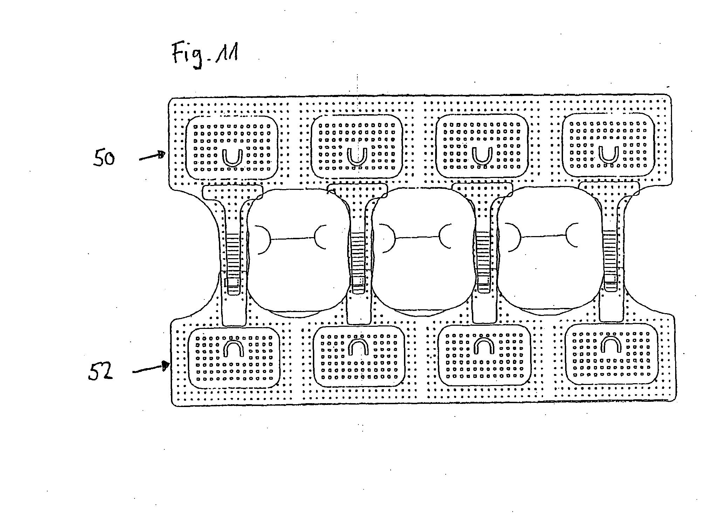

Figur 10 zeigt eine Membran der Figur 9 in zusammengeschobener Form zwischen zwei Zähnen 5B. Die Lasche 51 wurde durch die Durchtrittsöffnung 53 geführt und festgezogen bis die Membranteile 50,52 zirkulär eng und dicht an den Zähnen 58 anliegen.

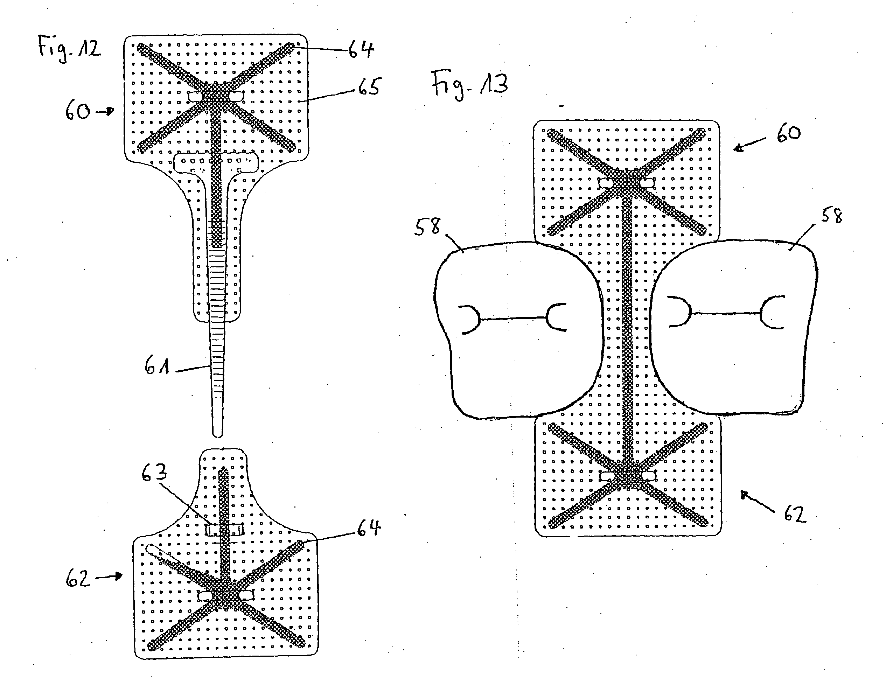

Figur 11 zeigt eine Ausführungsform der Membran der Figur 9 zwischen drei Zähnen 58. Die zwei Membranteile 50,52 sind je in einem Stück gefertigt, welche sich in vier Teile mit gleichem Aufbau wie die Membran der Figur 9 gliedern.

Figur 12 zeigt eine beispielhafte Membran mit Verstärkungsleisten. Die Membran besteht aus einem Teil 60 mit Lasche 61 und einem Teil 62 mit einer Durchtrittsöffnung 63 für die Lasche 61. Beide Teile 60,62 weisen Verstärkungsleisten 64 und Poren 65 auf. In einer bevorzugten Ausführungsform sind die Verstärkungsleisten 64 Titanlamellen, die in die Membran integriert sind. Sie gewähren anatomische Adaptation und Formstabilität und sind als Kraftanssatzpunkte für das Zugmittels 6 verwendbar. Das Zugmittel 6 kann z.B. durch einfaches Umstechen an den Titanlamellen befestigt werden. Unter Umstechen wird verstanden, dass die Membran durchstochen wird und das Zugmittel z.B. um eine Titanlamelle geführt wird.

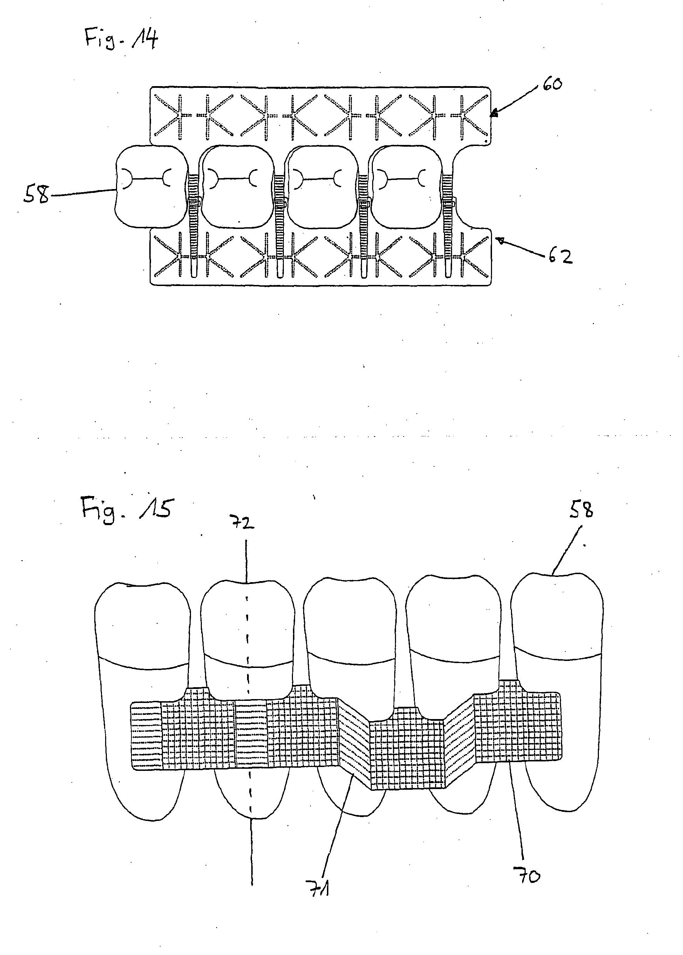

Figur 13 zeigt eine Membran der Figur 12 in zusammengeschobener Form zwischen zwei Zähnen 58. Die Lasche 61 wurde durch die Durchtrittsöffnung 63 geführt und festgezogen bis die Membranteile 60,62 zirkulär eng und dicht an den Zähnen 58 anliegen.

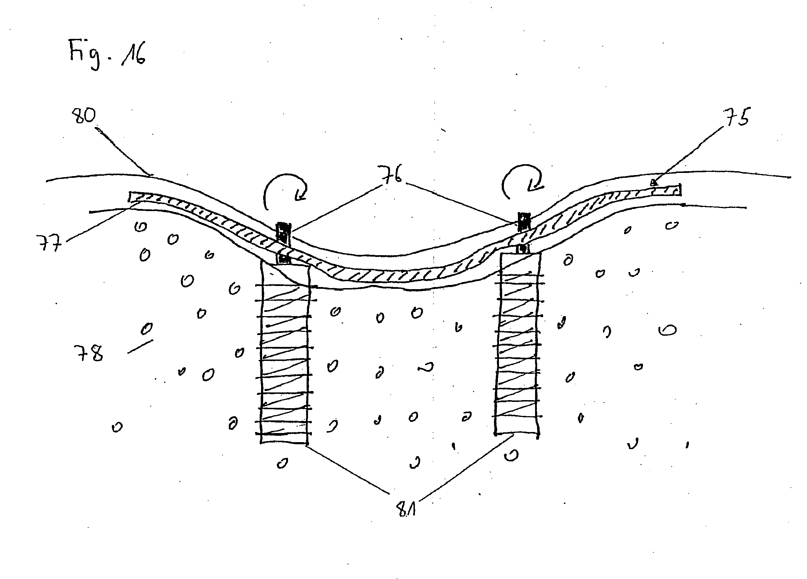

Figur 14 zeigt eine Ausführungsform der Membran der Figur 12 zwischen mehreren Zähnen 58. Die zwei-Membranteile 60,62 sind je in einem Stück gefertigt, welche sich in vier Teile mit gleichem Aufbau wie die Membran der Figur 12 gliedern.

Figur 15 zeigt eine beispielhafte Ausführungsform einer segmentierten Membran an Zähnen 58.

Die einzelnen Segmente 70 der Membran sind über Gelenke 71 miteinander verbunden. Als Gelenke sind Dehnungszonen bevorzugt, die die Applikation der Membran erleichtern und vertikales verschieben der Membran erlauben, sowie eine zirkuläre Abdeckung durch enges Anliegen an den Zähnen gewährleisten. Vorteilhaft sind die Gelenke 71 über einer Zahnlängsachse 72 angeordnet und ihre Knochen zugewandte Oberfläche entspricht dem Aufbau der erfindungsgemässen Membran.

Eine segmentierte Membran, wie oben beschrieben, erlaubt, dass mit einer Operation ein ganzer Kiefer auf einmal behandelbar ist. Bevorzugt sind Membraneinheiten, bei welchen der bukkale und linguale Teil interdental verbunden ist oder nachträglich leicht zusammenfügbar ist, um eine einfache Handhabung zu ermöglichen.

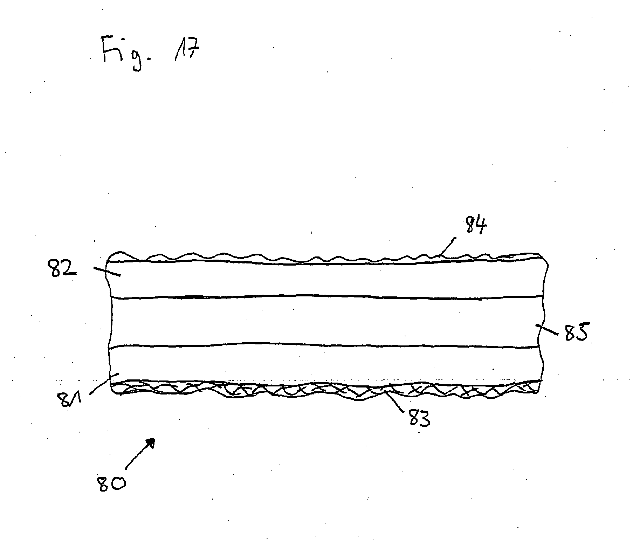

Figur 16 zeigt eine beispielhafte Anordnung zur Knochenregeneration, die mittels Anhebung einer erfingungsgemässen Membran durch ein Hebemittel z.B. provisorische Implantate, in dem unter der Membran liegenden Knochen Apposition induziert.

Die Anordnung umfasst eine erfindungsgemässe medizinische Membran 75, an welcher zwei beispielhafte Verstärkungspunkte 76 dargestellt sind. Die untere Oberfläche der Membran 77 ist mit dem Knochen 78 verbunden und ihre obere Oberfläche 79 ist von der Mundschleimhaut 80 bedeckt. Die Membran 75 ist über die Verstärkungspunkte 76 mit einem Hebemittel 81 verbunden. Die Hebemittel 81 sind so beschaffen, dass eine einstellbare Anhebung der Membran 75 möglich ist. Als Hebemittel sind z.B. provisorische Implantate mit Gewinde, die durch Drehung angehoben werden, verwendbar. Eine solche Ausführungsform eignet sich z.B. zur Knochenregeneration in einem Kiefergebiet, wo die Zähne durch Extraktion entfernt wurden. Bevorzugt werden die provisorischen Implantate nach erfolgter Knochenregeneration durch definitive Implantate ersetzt.

In der Figur 17 ist ein Querschnitt einer beispielhaften medizinischen Membran 80, die einen dreischichtigen Aufbau aufweist, dargestellt. Die dem Knochen zugewandte Schicht 81 und die dem Knochen abgewandte Schicht 82 bestehen aus nicht resorbierbarem Material z.B. Kunststoff. Die Oberfläche 83 der Knochen zugewandten Schicht 81 ist aufgerauht, um eine Verbindung mit dem darunterliegenden Knochen zu erlauben. Die Oberfläche 84 der Knochen abgewandten Schicht 82 ist aufgerauht, um ein Ablösen der Membran 80 vom bedeckenden Bindegewebe zu verhindern. Die mittlere Schicht 85 verleiht der Membran Formstabilität. Als erfindungsgemässe Membranen sind auch Membranen verwendbar, die einen einschichtigen, zweischichtigen oder mehrschichtigen Aufbau haben.

Eine erfindungsgemässe Membran zeichnet sich durch hohe Anwenderfreundlichkeit aus, da sie einem Patienten durch einen Operateur leicht einsetzbar ist. Bei der Anwendung an engstehenden Zähnen oder verblockten Kronen wird die Membran vorteilhaft in einem lingualen und bukkalen Teil hergestellt und erst durch den Operateur nach dem Einsetzen verbunden z.B. durch Zusammenschieben der beiden Teile.

Bei nicht engstehenden Zähnen, d.h. wenn keine engen approximalen Kontaktpunktverhältnisse vorliegen, wird vorteilhaft eine in einem Stück gefertigte Membran verwendet. Eine segmentierte Membran wird vorteilhaft bei der Anwendung an mehreren Zähnen eingesetzt, da ihre Gelenke, bevorzugt Dehnungszonen, eine genügende Beweglichkeit für die Applikation und eine Anpassung der Membran an unterschiedlichen vertikalen Knochenbau bei den einzelnen Zähnen erlauben.

Der Einsatz der Gegenstände der vorliegenden Erfindung ist nicht auf die Knochenregeneration im Zahnbereich beschränkt. Sie können auch z.B. für Kieferkammaufbau, in der Implantologie oder in der ästhetischen Gesichtschirurgie verwendet werden. Sie finden überall dort Verwendung, wo Knochenregeneration notwendig ist, aber nur kleine Befestigungsvorrichtungen einsetzbar sind.

Die Gegenstände der vorliegenden Erfindung sind auch in der Orthopädie und Traumatologie verwendbar, um vollständige Knochenregeneration von Röhrenknochen mittels periostaler Ossifikation zu erreichen.

Während in der vorliegenden Anmeldung bevorzugte Ausführungen der Erfindung beschrieben sind, ist klar darauf hinzuweisen, dass die Erfindung nicht auf diese Beschränkt ist und in auch anderer Weise innerhaldes Umfangs der folgenden Ansprüche ausgeführt werden kann.

## Patentansprüche

1. Verwendung einer medizinischen Membran zur Paradontalregeneration, wobei die Membran an mindestens einer ihrer Oberflächen mindestens ein Befestigungselement (55) für ein Zugmittel und/oder einen Verstärkungspunkt (76) besitzt.

2. Verwendung einer medizinischen Membran nach Anspruch 1, **dadurch gekennzeichnet, dass** die Membran Verstärkungsleisten (64) aufweist.

3. Verwendung einer medizinischen Membran nach einem der vorherigen Ansprüche, **dadurch gekennzeichnet, dass** die Membran formstabil ist.

4. Verwendung einer medizinischen Membran nach einem der vorherigen Ansprüche, **dadurch gekennzeichnet, dass** die Membran zur Kraftübertragung von einem Befestigungselement auf einen Knochen geeignet ist.

5. Verwendung einer medizinischen Membran nach einem der vorherigen Ansprüche, **dadurch gekennzeichnet, dass** die Membran eine dreidimensionale Fläche bildet.

6. Verwendung einer medizinischen Membran nach einem der vorherigen Ansprüche, **dadurch gekennzeichnet, dass** die Membran Segmente aufweist, die gelenkig miteinander verbunden sind.

7. Verwendung einer medizinischen Membran nach einem der vorherigen Ansprüche, **dadurch gekennzeichnet, dass** die Membran zumindest eine aufgeraute Oberfläche besitzt.

8. Verwendung einer medizinischen Membran nach einem der vorherigen Ansprüche, **dadurch gekennzeichnet, dass** beide Oberflächen der Membran aufgeraut sind.

9. Verwendung einer medizinischen Membran nach einem der vorherigen Ansprüche, **dadurch gekennzeichnet, dass** die aufgeraute Oberfläche aus Kollagen besteht.

10. Verwendung einer medizinischen Membran nach einem der vorherigen Ansprüche, **dadurch gekennzeichnet, dass** das Kollagen eine Maschenweite von 20 bis 100 µm aufweist.

11. Verwendung einer medizinischen Membran nach einem der vorherigen Ansprüche, **dadurch gekennzeichnet, dass** die Membran für Serum durchlässig ist.

12. Verwendung einer medizinischen Membran nach einem der vorherigen Ansprüche, **dadurch gekennzeichnet, dass** die Membran nicht resorbierbar ist.

13. Verwendung einer medizinischen Membran nach einem der Ansprüche 1 bis 11, **dadurch gekennzeichnet, dass** die Membran resorbierbar ist.

14. Verwendung einer medizinischen Membran nach Anspruch 13, **dadurch gekennzeichnet, dass** die resorbierbare Membran so beschaffen ist, dass ein Bestehen der Membran während einer Distraktions-, Retentions- und Konsolidierungsphase der Knochenregeneration gewährleistet ist.

15. Verwendung einer medizinischen Membran nach einem der vorherigen Ansprüche, **dadurch gekennzeichnet, dass** die Membran für Epithel- und Bindegewebszellen undurchlässig ist.

16. Verwendung einer medizinischen Membran nach einem der vorherigen Ansprüche, **dadurch gekennzeichnet, dass** die Membran aus mindestens zwei Teilen besteht, die über mindestens einen zur Einführung in einen interdentalen Zwischenraum geeigneten Steg verbunden sind, und insbesondere, dass der Steg eine Lasche aufweist, welche mit einem der Teile fest verbunden ist und in einer Öffnung des anderen der Teile eingreift.

17. Verwendung einer medizinischen Membran nach Anspruch 16, **dadurch gekennzeichnet, dass** die Teile über mehrere beabstandete Stege verbunden sind, derart, dass zwischen den Stegen mindestens eine Aufnahmeöffnung zur Aufnahme eines Zahnes gebildet wird.

18. Verwendung einer medizinischen Membran nach einem der vorherigen Ansprüche mit einer Zugbefestigungsvorrichtung, **dadurch gekennzeichnet, dass** die Zugbefestigungsvorrichtung ein Befestigungsmittel (10, 20), ein am Befestigungsmittel angeordnetes einstellbares Spannmittel (12, 22, 30, 40, 42) und ein am Spannmittel angeordnetes Zugmittel (6) zur Befestigung der Membran umfasst.

19. Verwendung einer medizinischen Membran nach Anspruch 18, **dadurch gekennzeichnet, dass** die Zugbefestigungsvorrichtung eine Grundplatte (10, 20) mit einer im Wesentlichen flachen Oberfläche (16, 27) zur adhäsiven Befestigung aufweist und dass insbesondere auf der Grundplatte das Spannmittel angeordnet ist.

20. Verwendung einer medizinischen Membran nach einem der vorherigen Ansprüche 18 oder 19, **dadurch gekennzeichnet, dass** das Spannmittel zur Aufwicklung des Zugmittels ausgestaltet ist.

21. Verwendung einer medizinischen Membran nach einem der Ansprüche 18 oder 19, **dadurch gekennzeichnet, dass** das Zugmittel über eine translatorische Bewegung eines Schiebers (30) spannbar ist.

22. Verwendung einer medizinischen Membran nach einem der vorherigen Ansprüche 18 bis 21, **dadurch gekennzeichnet, dass** das Zugmittel über mindestens ein Zahnrad gekoppelt ist.

23. Verwendung einer medizinischen Membran nach einem der vorherigen Ansprüche 18 bis 22, **dadurch gekennzeichnet, dass** die Zugbefestigungsvorrichtung für die Befestigung an einem Zahn geeignet ist.
